# EUROPEAN PATENT SPECIFICATION

(11) **EP 3 952 799 B1**
(45) Date of publication and mention of the grant of the patent: **26.03.2025**
(21) Application number: 20719694.0
(22) Date of filing: 08.04.2020
(51) Int. Cl.: A61F 2/95

(54) **MEDICAL DEVICE DELIVERY SYSTEM**
SYSTEM ZUR ABGABE EINER MEDIZINISCHEN VORRICHTUNG
SYSTÈME DE POSE DE DISPOSITIF MÉDICAL

(30) Priority: 08.04.2019 GB 201904922
(43) Date of publication of application: 16.02.2022
(73) Proprietor: Vascutek Limited, Renfrewshire PA4 9RR (GB)
(72) Inventor: SIMPSON, Graeme, Glasgow Central Scotland G11 5JB (GB)
(74) Representative: Murgitroyd & Company
(86) International application number: PCT/GB2020/050912
(87) International publication number: WO 2020/208349

(56) References cited:
- US-A1- 2013 289 703
- US-A1- 2014 236 278

## Description

The present invention relates to a medical device release apparatus.

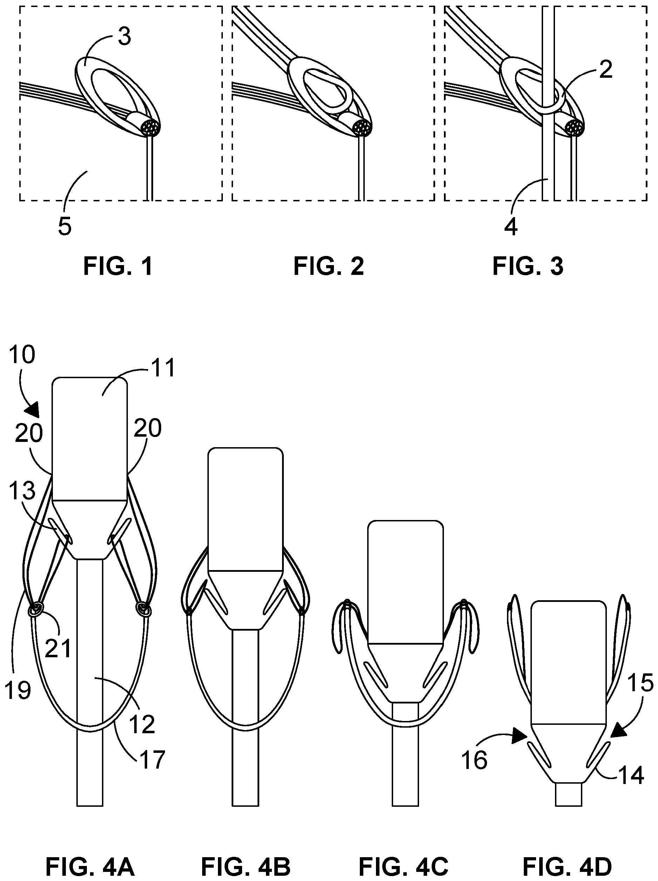

In this regard, medical devices such as endovascular stent devices are generally attached to their delivery system using a combination of control loop, eyelet and release wire. As shown in Figures 1 to 3, this connection is achieved by threading the free end of a control loop 2 of a control thread through an eyelet 3 with the other end being fixed to the tip of the delivery system. A release wire 4 is then passed through the open loop of the control thread, created when the control thread is passed through the eyelet, to prevent it from being pulled back through the eyelet. The medical device 5 is released from the delivery system by removing the release wire 4, which allows the control loop 2 to pull back through the eyelet, freeing the device from the delivery system.

This arrangement can cause issues if the release wire is damaged and cannot be removed from the control loop. This prevents the user from being able to remove the delivery system from the patient without additional bail out procedures or a full conversion to open surgery.

Issues can also occur if the user forgets to remove the release wire before attempting to remove the delivery system. This causes the device to migrate from the intended sealing zone, disrupting the seal and potentially damaging the vessel in the process.

US 2013/289703 A1 discloses a delivery system with capture fingers for delivering a stent-graft.

The present invention provides an apparatus, which seeks to alleviate the problems of such known arrangements. The invention is defined in independent claim 1. Certain optional features of the invention are defined in the dependent claims.

According to the present disclosure there is provided a medical device delivery apparatus, the apparatus comprising:- a head element at a proximal end of the apparatus, the head being configured for extending through the medical device and projecting proximally therefrom; the head element having one or more hook elements for engaging a control loop coupled to the medical device, wherein the one or more hook elements are axially fixed in relation to the head element.

In this manner, the medical device can be released at the same time as the delivery system is withdrawn without additional mechanical operation of the apparatus.

Preferably, the one or more hook elements are forward facing, with their free end extending in a direction towards the proximal end of the head element.

Conveniently, the head element has two hook elements.

Preferably, the hook elements are provided at diametrically opposite sides of the head element.

Conveniently, the one or more hook elements extend proximally to project radially outwardly from the longitudinal profile of the head element.

Preferably, the one or more hook elements are provided at a distal end of the head element.

Conveniently, the one or more hook elements each take the form of an inclined slot, open at a proximal end and closed at a distal end.

Preferably, each slot has a tapering profile.

Conveniently, the head element comprises a head body and a head plate, the head plate being coupled to a distal end of the head body to define said one or more hook elements between the head body and the head plate.

Preferably, the head body is chamfered at its distal end, to define said one or more hook elements.

Conveniently, the apparatus further comprises one or more attachment points for an end of a control loop.

Preferably, the one or more attachment points are provided on the head element proximally of the one or more hook elements.

Conveniently, the one or more hook elements are moveable between deployed and un-deployed positions.

Preferably, the one or more hook elements are pivotable between the deployed and un-deployed positions.

Conveniently, the one or more hook elements are retractable so as to be substantially flush with the longitudinal profile of the apparatus.

Preferably, the one or more hook elements are profiled so that rotation of the head element promotes release of any control loop engaged by the one or more hook element.

Conveniently, the one or more hook elements comprise an angled section having a radially extending portion, the radially extending portion being configured to engage a control loop.

The apparatus preferably further comprises alignment means for aligning said one or more hook elements with one or more eyelets of medical device.

According to a further aspect of the present disclosure there is provided a medical device deployment system for deploying a medical device comprising:- medical device delivery apparatus having a head element at a proximal end of the apparatus, the head being configured for extending through the medical device and projecting proximally therefrom, the head element having one or more hook elements for engaging a control loop coupled to the medical device, wherein the one or more hook elements are axially fixed in relation to the head element.

According to a yet further aspect of the present disclosure not forming part of the invention there is provided a method of deploying a medical device using the delivery apparatus or deployment system of any preceding claim, the method comprising the steps:- passing said delivery apparatus through the interior of the medical device so that it protrudes from a proximal end thereof; coupling the medical device and one or more hook elements of the delivery apparatus using one or more control loops; advancing the apparatus to the deployment site; deploying the medical device at the site; and retracting the delivery apparatus, the one or more control loops releasing from the one or more hook elements, wherein the one or more hook elements are axially fixed in relation to the head element.

Preferably, said one or more control loops are attached to the head element of the delivery apparatus, pass through one or more eyelets provided to said medical device and then pass around said one or more hook elements before passing again through the one or more eyelets.

Conveniently, the one or more control loops are attached to the medical device, and pass around the one or more hook elements.

Preferably, the medical device has an undulating proximal end profile, the one or more control loops being attached to proximal peaks of the medical device.

Conveniently, the one or more hook elements are moved to facilitate release the one or more control loops.

Preferably, the one or more hook elements are pivoted rearwardly to facilitate release the one or more control loops.

Conveniently, the head element is rotated about its longitudinal axis to facilitate release the more or more control loops.

Embodiments of the present disclosure will now be described by way of example and with reference to the accompanying drawings, of which:-
Figures 1 to 3 show a known arrangement for delivering a medical device to a site;
Figures 4a to 4d show a first embodiment of the present disclosure;
Figure 5a to 5e show a second embodiment of the present disclosure;
Figure 6a to 6c show a third embodiment of the present disclosure; and
Figures 7a and 7b show a fourth embodiment of the present disclosure.

Figures 1 to 3 are discussed above and relate to a conventional delivery system for an endovascular device. Such systems can experience issues where the release wire is damaged or where the user forgets to remove it before attempting to remove the delivery system.

The present invention hence seeks to provide a medical device delivery system that does away with the need for release wire.

In this respect, as shown in Figures 4a to 4d, the delivery apparatus 10 comprises a tip or head element 11 at the proximal end of a longitudinal shaft 12. The head element has hook elements 13 that project radially outwardly and forwardly towards the proximal end of the head element. The hook elements may be formed in any suitable manner, and in the present embodiment are formed by chamfering the distal end of the head element and coupling a plate 14 thereto, in order to form slots 15. As shown, the ends 16 of the plates preferably project radially outwardly of the general outer longitudinal profile of the head element 11.

As shown, in use the head element is positioned to extend through the interior of a medical device 17, such as an endovascular stent, so that the head element 11 projects out of its proximal end. For clarity, the structure of the medical device is not shown in detail in the figures but may comprise an expandable stent device having an undulating proximal end denoted by reference 17. In the embodiment of Figures 4a to 4d, control loops 19 are attached at one end to the head element at attachment points 20 above the hook elements, namely closer to the proximal end of the head element.

Each control loop extends rearwardly and passes through an eyelet 21 coupled to the medical device. The loop then returns back to the head element to be looped around hook element 11 before returning to the eyelet and then up towards the proximal end of the apparatus.

Figures 4a to 4d show the progression of the head element relative to the medical device 17. In Figure 4a, the medical device has been moved to the correct position in relation to the site where it can be fixed in position. This may be by expanding the device so that it seals against the internal surfaces of a vessel in which it is being deployed.

Once correctly fixed in position, the delivery apparatus is retracted as shown in Figures 4b to 4d so that it passes rearwardly within the device towards its distal end.

As the eyelets become level with the hook elements 11, the control loops are no longer pulled downwardly into the hook elements and can therefore start to become free of the hook elements, as shown in Figures 4b and 4c. Then at Figure 4d the control loops are no longer hooked around the hook elements so can run through the eyelets as the delivery apparatus is retracted.

The head element design removes the requirement for release wires in the attachment method and releases the device at the same time as the delivery system is removed from the patient, with no additional actions required from the user.

As described above, this may be achieved this by increasing the length of the control loops and incorporating two hooks, one at either side of the head element or tip, colinear with the control loops. To assemble the device to the delivery system the control loops are fed through the eyelets before the free ends are attached to the hooks on the tip.

When the device eyelets are lower than the tip, the control loops will stay attached to the hooks. The device will therefore stay attached to the delivery system during compaction and unsheathing. However, when the tip is moved lower than the eyelets (e.g. when the delivery system is removed from the device), the control loops will detach from the hooks, allowing them to pull out of the eyelets and freeing the device from the delivery system.

The configuration of the present disclosure as such means that a separate mechanical step is not required as the apparatus is withdrawn. The device is released merely by withdrawing the head element, i.e. the delivery system tip.

In this connection, the hooks elements are axially fixed in place on the delivery system tip, and it is the relative position of the graft, with respect to the hooks, that fixes or releases the device.

In this connection, the hooks elements/slots should preferably be:- integrated onto the delivery system; above the level of the device; angled towards the device so that the 'closed' portion of the hook/slot is closest to the device; and open at one end so the control loops are free to leave. The control loops are preferably:- attached to the delivery system at one end (with the other end free); and long enough to loop through the eyelets and attach onto the hooks. The eyelets preferably are attached to the device.

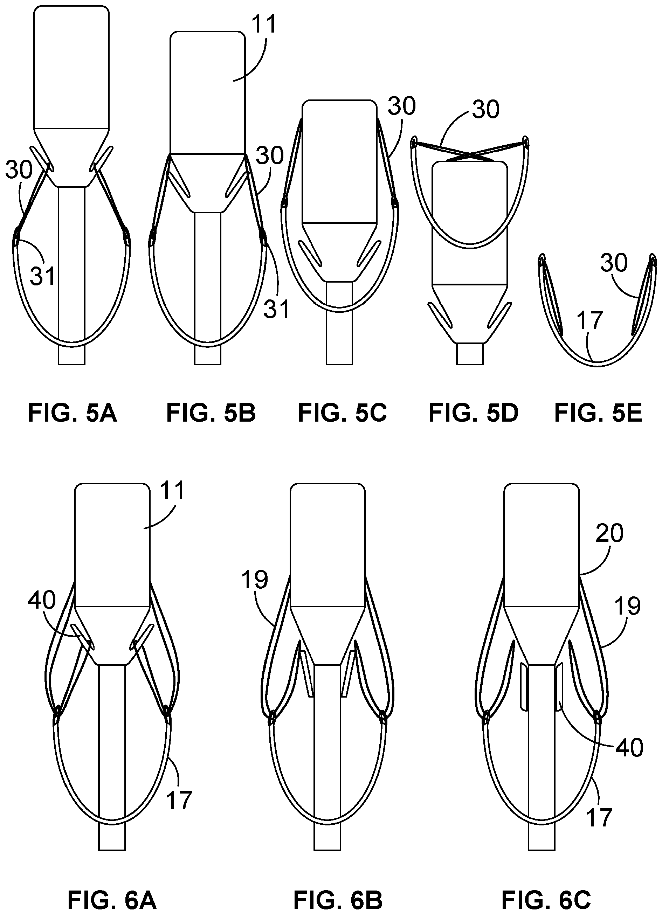

A second embodiment of the present disclosure not forming part of the invention is described in relation to Figure 5a to 5e.

In this embodiment, control loops 30 are attached to the peaks 31 of the 1st ring of the medical device. When assembling the device to the delivery system, the control loops simply latch onto the hook elements 32 provided on the tip. This attachment method functions in the same way as above in relation to the first embodiment (the relative position of the tip with regard to the control loop attachment point would dictate whether or not the device was attached to the delivery system). However, this embodiment would leave control loops attached to the device in situ.

The hook elements may be provided with a secondary feature that allows them to be removed from the head element or tip, leaving the control loops free to pull out of the eyelets. This would aid in any circumstance in which the control loops became caught on the hook elements preventing the device from releasing.

Figures 6a to 6c shows a third embodiment of the present disclosure where the hook elements 40 are able to be retracted in order to release the device.

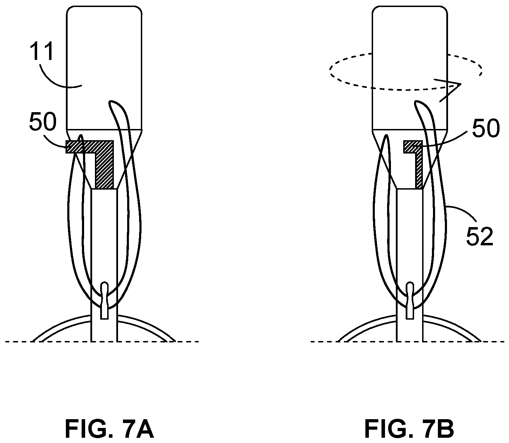

As shown in the fourth embodiment of the present disclosure of figures 7a and 7b, the hook elements 50 may be angled or shaped such that rotation of the head element 11 also causes or facilitates the control loops to exit the hooks.

The control loops 52 may run the length of the delivery system so that they can be cut and removed, freeing the device from the delivery system. This would act as a failsafe if the control loops got caught in the hook elements.

The various embodiments of the present disclosure maintain the advantages of tethering the top of the medical device to the delivery system for more accurate deployment and easier manufacturing, but they remove any additional steps required by the user to detach the device from the delivery system, such as removing release wires, top cap, etc. associated with known methods.

## Claims

1. A medical device delivery system (10), comprising: -
a) a longitudinal shaft (12) having a proximal end;
b) a head element (11) at the proximal end of the longitudinal shaft (12), the head element (11) having
i) a proximal end,
ii) a distal end, and
iii) one or more hook elements (13) extending proximally from the distal end of the head element (11);
c) the medical device (17) having a proximal end and one or more eyelets (21) at the proximal end of the medical device (17);
d) one or more control loops (19), each of the one or more control loops (19) extending through one of the one or more eyelets (21) and passing around one of the one or more hook elements (13), whereby, when the head element (11) is retracted and the one or more eyelets (21) become level with the one or more hook elements (13), the one or more control loops (19) are no longer pulled downwardly into the one or more hook elements (13) so that the one or more control loops (19) are no longer hooked around the one or more hook elements (13) and can run through the one or more eyelets (21) as the head element (11) is retracted, **characterized in that** the one or more control loops (19) are fixed to the head element (11) at a point on the head element (11) that is proximal to the one or more hook elements (13).

2. The medical device delivery system (10) of claim 1, wherein the head element (11) has two hook elements (13), and wherein the hook elements (13) are at diametrically opposite sides of the head element (11).

3. The medical device delivery system (10) of claim 1, wherein the one or more hook elements (13) extend radially outwardly from the head element (11).

4. The medical device delivery system (10) of claim 1, wherein the one or more hook elements (13) each take the form of an inclined slot, wherein each slot has a tapering profile.

## Patentansprüche

1. Ein System (10) zum Instellungbringen einer medizinischen Vorrichtung, das Folgendes beinhaltet:
a) einen longitudinalen Schaft (12), der ein proximales Ende aufweist;
b) ein Kopfelement (11) an dem proximalen Ende des longitudinalen Schafts (12), wobei das Kopfelement (11) Folgendes aufweist:
i) ein proximales Ende,
ii) ein distales Ende, und
iii) ein oder mehrere Hakenelemente (13), die sich proximal von dem distalen Ende des Kopfelements (11) erstrecken;
c) wobei die medizinische Vorrichtung (17) ein proximales Ende und eine oder mehrere Ösen (21) an dem proximalen Ende der medizinischen Vorrichtung (17) aufweist;
d) eine oder mehrere Steuerschlaufen (19), wobei sich jede der einen oder der mehreren Steuerschlaufen (19) durch eine der einen oder der mehreren Ösen (21) erstreckt und um eines des einen oder der mehreren Hakenelemente (13) herum verläuft, wodurch, wenn das Kopfelement (11) eingezogen wird und die eine oder die mehreren Ösen (21) mit dem einen oder den mehreren Hakenelementen (13) auf die gleiche Höhe kommen, die eine oder die mehreren Steuerschlaufen (19) nicht länger nach unten in das eine oder die mehreren Hakenelemente (13) gezogen werden, sodass die eine oder die mehreren Steuerschlaufen (19) nicht länger um das eine oder die mehreren Hakenelemente (13) herum gehakt werden und durch die eine oder die mehreren Ösen (21) verlaufen können, während das Kopfelement (11) eingezogen wird, **dadurch gekennzeichnet, dass** die eine oder die mehreren Steuerschlaufen (19) an dem Kopfelement (11) an einem Punkt auf dem Kopfelement (11) fixiert sind, der sich proximal zu dem einen oder den mehreren Hakenelementen (13) befindet.

2. System (10) zum Instellungbringen einer medizinischen Vorrichtung gemäß Anspruch 1, wobei das Kopfelement (11) zwei Hakenelemente (13) aufweist, und wobei sich die Hakenelemente (13) an diametral entgegengesetzten Seiten des Kopfelements (11) befinden.

3. System (10) zum Instellungbringen einer medizinischen Vorrichtung gemäß Anspruch 1, wobei sich das eine oder die mehreren Hakenelemente (13) von dem Kopfelement (11) radial nach außen erstrecken.

4. System (10) zum Instellungbringen einer medizinischen Vorrichtung gemäß Anspruch 1, wobei das eine oder die mehreren Hakenelemente (13) jeweils die Form eines schrägen Schlitzes annehmen, wobei jeder Schlitz ein sich verjüngendes Profil aufweist.

## Revendications

1. Un système de mise en place de dispositif médical (10), comprenant :
a) un arbre longitudinal (12) ayant une extrémité proximale ;
b) un élément formant tête (11) à l'extrémité proximale de l'arbre longitudinal (12), l'élément formant tête (11) ayant
i) une extrémité proximale,
ii) une extrémité distale, et
iii) un ou plusieurs éléments formant crochets (13) s'étendant proximalement depuis l'extrémité distale de l'élément formant tête (11) ;
c) le dispositif médical (17) qui a une extrémité proximale et un ou plusieurs œillets (21) à l'extrémité proximale du dispositif médical (17) ;
d) une ou plusieurs boucles de commande (19), chacune des une ou plusieurs boucles de commande (19) s'étendant à travers un des un ou plusieurs œillets (21) et passant autour d'un des un ou plusieurs éléments formant crochets (13), moyennant quoi, lorsque l'élément formant tête (11) est rétracté et que les un ou plusieurs œillets en viennent à être de niveau avec les un ou plusieurs éléments formant crochets (13), les une ou plusieurs boucles de commande (19) ne sont plus tirées vers le bas dans les un ou plusieurs éléments formant crochets (13) de sorte que les une ou plusieurs boucles de commande (19) ne sont plus accrochées autour des un ou plusieurs éléments formant crochets (13) et peuvent traverser les un ou plusieurs œillets tandis que l'élément formant tête (11) est rétracté, **caractérisé en ce que** les une ou plusieurs boucles de commande (19) sont fixées à l'élément formant tête (11) à un point sur l'élément formant tête (11) qui est proximal aux un ou plusieurs éléments formant crochets (13).

2. Le système de mise en place de dispositif médical (10) de la revendication 1, où l'élément formant tête (11) a deux éléments formant crochets (13), et où les éléments formant crochets (13) sont sur des côtés diamétralement opposés de l'élément formant tête (11).

3. Le système de mise en place de dispositif médical (10) de la revendication 1, où les un ou plusieurs éléments formant crochets (13) s'étendent radialement vers l'extérieur depuis l'élément formant tête (11).

4. Le système de mise en place de dispositif médical (10) de la revendication 1, où les un ou plusieurs éléments formant crochets (13) prennent chacun la forme d'une encoche inclinée, chaque encoche ayant un profil qui va en s'effilant.
